# EUROPEAN PATENT SPECIFICATION

(11) **EP 1 363 678 B1**
(45) Date of publication and mention of the grant of the patent: **18.05.2005**
(21) Application number: 01913053.3
(22) Date of filing: 26.02.2001
(51) Int. Cl.: A61L 9/02, A61L 9/03, A01M 1/20, A01N 25/20, A01G 13/06

(54) **COMPOSITION FOR AROMA DELIVERY WITH IMPROVED STABILITY AND REDUCED FOAMING**
AROMA FREISETZENDES MITTEL MIT VERBESSERTER STABILITÄT UND VERMINDERTER SCHAUMBILDUNG
COMPOSITION CONNUE POUR DEGAGER UN AROME, A STABILITE AMELIORE ET A MOUSSAGE REDUIT

(43) Date of publication of application: 26.11.2003
(73) Proprietor: THE PROCTER & GAMBLE COMPANY, Cincinnati, Ohio 45202 (US)
(72) Inventor: LI, Yujun, Kobe, Hyogo 658-0032 (JP)
(74) Representative: Canonici, Jean-Jacques
(86) International application number: PCT/US2001/006092
(87) International publication number: WO 2002/068005

(56) References cited:
- WO-A-99/48469
- WO-A-99/48539
- US-A- 3 535 246
- US-A- 3 903 011
- US-A- 5 935 486
- PATENT ABSTRACTS OF JAPAN vol. 018, no. 433 (C-1237), 12 August 1994 (1994-08-12) & JP 06 134025 A (SUNSTAR INC), 17 May 1994 (1994-05-17)
- DATABASE WPI Section Ch, Week 198733 Derwent Publications Ltd., London, GB; Class A96, AN 1987-230747 XP002181610 & JP 62 153216 A (JAPAN EBIAN YG), 8 July 1987 (1987-07-08)

## Description

### TECHNICAL FIELD

The present invention relates to continuous phase reaction mixtures with improved stability and aesthetics that include exothermic generating particles having a water soluble coating that encases all of the particles, a volatile component, an anti-foaming agent and a buffer. The reaction mixtures are especially suited to generate heat in a controllable manner. When mixed with an aqueous solution, volatile components can be controllably released to the surrounding environment by the present reaction mixtures. Apparatuses and methods that use these reaction mixtures are also disclosed.

### BACKGROUND OF THE INVENTION

There are many methods for delivering airborne components, such as fragrances, insect repellents and the like. Scented candles, for example, are well known implements for delivering a desirable smell to the air. Incense performs essentially the same function, but the aroma is typically the natural smell evolved when the incense is burned. That is, incense typically does not require the addition of a fragrant component, while scented candles are generally a mixture of wax and a fragrance. In yet another variant of aroma delivering combustion devices, candles have been used to heat liquids or gels causing a volatile component to evolve. Moreover, lamps that burn oil have been used for ages, not only to provide light, but also to deliver fragrances. Combustion devices for delivering fragrances are well known, but most of these devices have also been used to deliver other airborne components, such as insect repellents, medicinal vapors such as eucalyptus, and other compounds.

Unfortunately, combustion devices inherently give rise to safety issues. They can be accidentally knocked over resulting in a fire, or when left unattended, many combustion devices can burn down to their base and ignite the surrounding surface. Moreover, smoke is an inevitable by-product of any combustion device. In general, smoke from a combustion device can be noxious, and may cause long-term health problems. Thus, while these devices are simple and inexpensive methods for delivering airborne components, they are not without problems.

Another method of delivering airborne components is to simply rely on evaporation. For example, a liquid, solid or gel material that contains an airborne component can be placed anywhere and over time the airborne component will evolve to the surrounding environment via evaporation. But this system relies on the difference between the vapor pressure of the airborne component and atmospheric pressure. If the vapor pressure of the airborne component is too high, the component will be delivered too fast. Likewise, if the vapor pressure of the component is too low, the component will be delivered too slowly to make a marked effect in the surrounding environment. Many insect repellents, for example, cannot be delivered effectively by evaporation alone because of their high vapor pressure. Thus, evaporative devices are very limited in the type of material they can deliver, and the speed with which these select materials can be delivered.

Slightly more advanced apparatuses for delivering airborne components use electrical power from batteries or an electrical outlet in the home. These devices typically use the electricity to provide heat, forced airflow, or both to speed the delivery of the airborne component. Unfortunately, these devices are necessarily more complicated and expensive to build and operate than are combustion and evaporative devices. While these devices may improve delivery, they increase complexity and cost. Moreover, the devices that are not battery operated are inherently not portable, as they require an electrical outlet.

Sprays and aerosols can deliver a wide variety of materials to the air. But these devices are, in general, manually operated and provide a short burst of the delivered component. Sprays and aerosols are not well suited for the prolonged delivery of a substance unless they are provided with a mechanical control mechanism. Such mechanical controls are expensive and limit the portability of such devices.

Self-contained exothermic reaction mixtures that are initiated by the addition of an aqueous solution have been considered for delivering compositions to the surrounding air. A self-contained exothermic reaction can provide heat without combustion or electrical source. The heat, in turn, can speed the evaporation of the composition that one wishes to deliver. As such, a wider range composition can be delivered in this manner. But these reactions have one substantial problem, they are hard to control. For example, it has been difficult to design a reaction system that is self-contained, and runs at a constant temperature for an extended period of time. It is axiomatic that one cannot control the delivery of the desired composition without controlling the temperature of the reaction system.

Commonly assigned, co-pending U.S. patent applications, serial no. 00/19079, filed July 13, 2000, entitled "Methods And Reaction Mixtures For Controlling Exothermic Reactions"; commonly assigned, co-pending U.S. patent application serial no. 00/19081, filed July 13, 2000, entitled "Multi-Layer Reaction Mixtures And Apparatuses For Delivering A Volatile Component Via A Controlled Exothermic Reaction"; and commonly assigned, co-pending U.S. patent application, serial no. 00/19080, filed July 13, 2000, entitled "Methods And Apparatuses For Delivering A Volatile Component Via A Controlled Exothermic Reaction," all being incorporated herein by reference, disclose a system using water soluble coatings for a portion of the exothermic particles control the reaction rate of the exothermic reaction. However, the present invention provides substantial improvements over these earlier disclosures. There still exists a need for a reaction mixture that eliminates the migration of exothermic particles within the water soluble coating and diminishes the presence of foam during the exothermic reaction.

### SUMMARY OF THE INVENTION

The present invention is directed to a continuous phase reaction mixture comprising the following reaction components: exothermic generating particles comprising a water soluble coating that encases all of the particles, with the water soluble coating comprised of polyethylene glycol (PEG) having a molecular weight from 2000-6000, and mixtures thereof, a buffer, an anti-foaming agent and a volatile component. Optionally, the reaction components further comprise an optional component selected from the group consisting of a thickening agent, an aqueous solution, or a mixture thereof.

In one aspect of this invention, the reaction components are mixed together, and the temperature of the reaction mixture increases to a Set Temperature that is greater than about 35°C and less than about 75°C within less than 20 minutes. More preferably, the reaction mixture remains within 10°C of the Set Temperature for at least about 45 minutes, preferably for at least about 60 minutes, most preferably for at least about 80 minutes. The present invention has a continuous phase where the water soluble coating encases the reaction components. The continuous phase provides improved storage stability while still obtaining the desired properties as discussed above.

The exothermic generating particles of the present invention are preferably selected from the group consisting of uncomplexed metals, metal salts, metal oxides, metal hydroxides, metal hydrides and mixtures thereof. The metals are selected from the group consisting of beryllium, magnesium, lithium, sodium, calcium, potassium, iron, copper, zinc, aluminum and mixtures thereof.

There is further provided in the present invention a process for generating heat comprising the steps of: providing exothermic generating particles entirely encased by a water soluble coating, such water soluble coating comprising at least one water soluble coating ingredient consisting of PEG having a molecular weight from 2000-6000, and mixtures thereof; providing a buffer, an anti-foaming agent, a volatile component, with any optional components and adding the combination into an aqueous solution.

In yet another aspect of this invention there is provided an apparatus for generating heat comprising a container and the following reaction components: exothermic generating particles entirely encased by a water soluble coating, such water soluble coating comprising at least one water soluble coating ingredient consisting of PEG having a molecular weight from 2000-6000, and mixtures thereof; a volatile component, a buffer, an anti-foaming agent, an aqueous solution, and optionally a thickening agent.

The first improvement of the present invention is to provide a continuous layer system that prevents separation of the water soluble coating and the exothermic particles when stored more than 24 hours to provide a quick starting reaction time (i.e. less than 20 minutes) after storage. The present invention accomplishes these improvements by increasing the molecular weight of the PEG used as the water soluble coating.

The second improvement of the present invention is to reduce the presence of foaming of the exothermic reaction composition through the addition of an anti-foaming agent. In previous exothermic reaction compositions, the foaming height could reach up to 15 mm during the use of the exothermic reaction composition resulting in a negative aesthetic appearance.

The methods and apparatuses of this invention provide portable and inexpensive ways to deliver compositions to the surrounding air in a controllable manner. The devices can be relatively small while operating in a controllable manner for an extended period of time. For example, a reaction mixture can be designed to deliver a component to the surrounding environment for an extended period of time at a relatively controlled rate.

The apparatuses of this invention can be used to deliver a variety of useful compounds to the surrounding air, and to clothes, carpet, pets, skin and many other surfaces. Moreover, the apparatuses of this invention can be combined with color and light to improve the aesthetic qualities, and ultimately, improve the overall experience for the user of the apparatus.

### BRIEF DESCRIPTION OF THE DRAWINGS

While the specification concludes with claims particularly pointing out and distinctly claiming the invention, it is believed that the invention will be better understood from the following description of preferred embodiments which is taken in conjunction with the accompanying drawings in which:
Fig. 1 is a graphical representation of three controlled reactions with a Set Temperature of about 50°C using reaction mixtures according to the present invention, and an uncontrolled reaction; and
Fig. 2 is a schematic representation of an apparatus according to the present invention.

### DETAILED DESCRIPTION OF THE INVENTION

As noted, the present invention is directed to an improved reaction mixture comprising the following reaction components: exothermic generating particles comprising a water soluble coating of PEG with a molecular weight between 2000 and 6000, and mixtures thereof, that entirely encases the particles; a buffer, an anti-foaming agent and a volatile component. Optionally, the reaction components further comprise a thickening agent, an aqueous solution, or a mixture thereof. The reaction mixture can be used to generate heat in a controllable manner, which, in turn, assists in the evolution of the volatile component in a controlled manner. Apparatuses that utilize the reaction mixtures taught herein are also disclosed.

"Continuous phase" as used herein, is defined to mean that there is one visible layer through the reaction mixture. This being distinct from a multi-phase reaction mixture where there is visibly distinct stratification of two or more layers.

### Reaction Mixture

In one aspect of this invention a continuous phase reaction mixture is formed by mixing the reaction components that are suspended in a water soluble coating to initiate an exothermic reaction between the exothermic generating particles and the aqueous solution. The exothermic reaction generates heat, which elevates the temperature of the reaction mixture. The heat, more precisely, the elevated temperature of the reaction mixture, aids the evolution of the volatile component from the reaction mixture. As will be understood, the water soluble coating of the exothermic generating particles, described below, can be used to control the speed of the exothermic reaction, and the heat generated. The ability to control the amount of heat generated by the reaction mixture, without any external controls, allows for the controlled delivery of the volatile component.

As is well known to those skilled in the art, chemical reactions can be difficult to control. Assuming a batch process, and putting aside thermodynamic considerations, the rate of an exothermic chemical reaction depends largely on the temperature and concentration of the reaction mixture. With no external controls, the temperature of an exothermic reaction mixture will rapidly increase during the early stages of the reaction. This is due largely to two factors, the concentration of the reactants is at its highest level, and as the reaction progresses heat is generated raising the temperature of the reaction mixture, which, in turn, increases the rate of the reaction. As the reactants are depleted, the reaction stows, causing a precipitous decrease in the temperature of the reaction mixture.

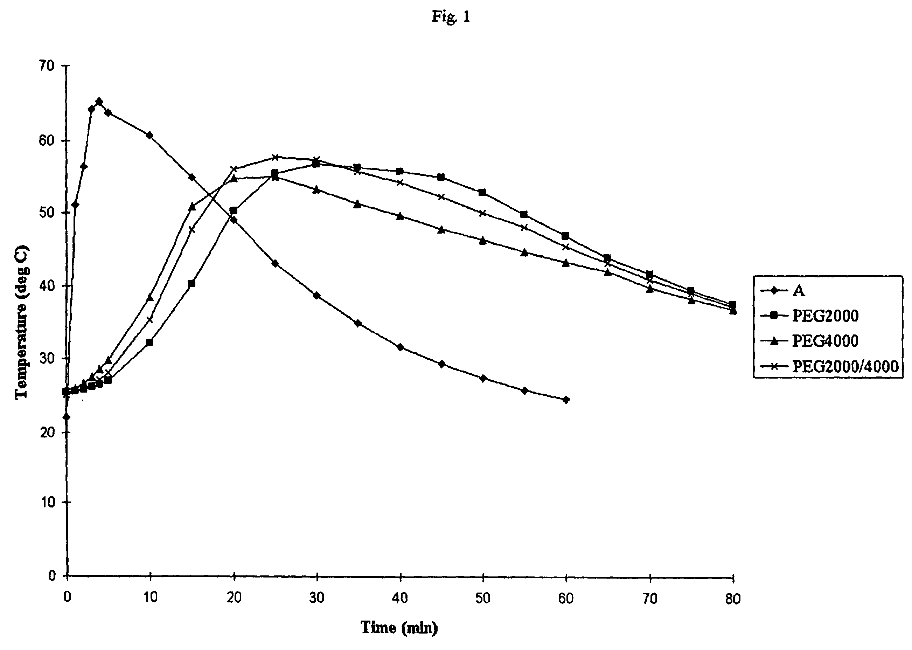

This effect is graphically illustrated in Figure 1, specifically; line "A" illustrates the temperature of an uncontrolled exothermic reaction mixture as a function of time. Figure 1 is discussed in greater detail below, but it clearly illustrates one problem addressed by the present invention. That is, the temperature of the reactions represented by Line "A" of Figure 1 varies considerably and terminates relatively quickly. Moreover, the rate of change of the temperature is almost never constant.

By coating the exothermic generating particles with the water soluble coating, as described in detail below, an exothermic reaction mixture can be designed to provide controlled heat over relatively long periods of time. And other control schemes can be easily designed by those skilled in the art, for example, a reaction mixture can be designed where the temperature increases gradually and a target temperature range is maintained for a relatively long period of time. Other control schemes will be apparent based on the following details.

In one such control scheme, a reaction mixture is prepared by mixing the reaction components to initiate an exothermic reaction. The temperature of the reaction mixture increases to a Set Temperature that is greater than about 35°C and less than about 75°C, preferably between about 35°C and 60°C, within less than about 30 minutes, preferably within less than about 20 minutes. Preferably, the reaction mixture remains within 15°C, more preferably within 10°C of the Set Temperature for at least about 45 minutes, preferably for at least about 60 minutes, and most preferably for at least about 80 minutes. It is understood that the term "remains within" as used herein, means the same as "±". For example, to "remain within 10°C" of a Set Temperature of 50°C, means the temperature can fluctuate between 40°C and 60°C. This control scheme is graphically illustrated in Figure 1 by Lines "PEG2000", "PEG4000", and "PEG2000/4000".

Figure 1 displays one "uncontrolled" exothermic reaction according to the prior art ("A") compared to three "controlled" reactions according to the present invention ("PEG2000", "PEG4000", and "PEG2000/4000"). The reaction components, and the resulting reaction mixture are given in Table 1 and summarized in Table 2. As can be seen, magnesium powder is used as the exothermic generating particles, and a citric acid buffer is used. The exothermic generating particles of reaction mixture "A" are uncoated while the exothermic generating particles of reaction mixtures "PEG2000", "PEG4000", and "PEG2000/4000" include exothermic particles coated with polyethylene glycol of different molecular weights.

**Table 1**

| | A | PEG 2000 | PEG 4000 | PEG 2000/4000 |
|---|---|---|---|---|
| | Wt. % | Wt. % | Wt. % | Wt. % |
| PEG 2000 | 0.0 | 19.10 | 0.0 | 9.55 |
| PEG 4000 | 0.0 | 0.0 | 19.10 | 9.55 |
| Magnesium | 13.3 | 10.57 | 10.57 | 10.57 |
| Citric acid | 86.7 | 68.92 | 68.92 | 68.92 |
| Perfume | 0.0 | 1.41 | 1.41 | 1.41 |
| Total | 100 | 100 | 100 | 100 |

**Table 2**

| | A | PEG 2000 | PEG 4000 | PEG 2000/4000 |
|---|---|---|---|---|
| | Wt. (g) | Wt. (g) | Wt. (g) | Wt. (g) |
| Coating | 0.0 | 2.71 | 2.71 | 2.71 |
| Mg | 2.6 | 1.50 | 1.50 | 1.50 |
| Citric Acid | 16.6 | 9.78 | 9.78 | 9.78 |
| Perfume | 0.0 | 0.20 | 0.20 | 0.20 |
| Total | 19.2 | 14.19 | 14.19 | 14.19 |

The ratio of exothermic particles to buffer was kept at roughly 1:6.5 (w/w) for each reaction mixture. The amount of exothermic particles and buffer were increased for "A" to more clearly show a typical uncontrolled exothermic reaction. "A" was added to 100.0 grams of water and "PEG2000", "PEG4000", and "PEG2000/4000" were added to 55.0 grams of water. As discussed briefly above, Line "A" is a typical graph of temperature verses time for an uncontrolled exothermic reaction. The temperature rises rapidly at first to a maximum of greater than 65°C. And then, as the reaction components are consumed, the temperature begins to decrease along a logarithmic curve. And within approximately 35 minutes, the reaction has cooled to within 5°C of the initial temperature (room temperature).

In sharp contrast, the reaction mixtures represented by lines "PEG2000", "PEG4000", and "PEG2000/4000"of Figure 1, increase to the Set Temperature of about 50°C within about 20 minutes. The reaction temperatures then remain within 10°C of the Set Temperature for at least about 80 minutes.

It is understood that the control scheme depicted in Figure 1, that is, where the reaction mixture rises to a Set Temperature and the temperature remains relatively constant for an extended period of time, is only one of many possible control schemes covered by the present invention.

### Reaction Components

Turning now to the reaction components, which include exothermic generating particles entirely encased by a water soluble coating, such water soluble coating comprising at least one water soluble coating ingredient consisting of PEG having a molecular weight from 2000-6000, and mixtures thereof; a volatile component, an anti-foaming agent, and a buffer. Preferably, the reaction components further comprise, a thickening agent and an aqueous solution, or a mixture thereof.

### Anti-foaming Agent

The present invention provides an improvement in that it reduces the appearance of foam at the top of a reaction mixture that has been stored upon the addition of an aqueous solution. Without being limited by a theory, it is believed that during storage a chemical degradation of volatile components occurs in the exothermic reaction mixture that results in the presence of foam during the reaction of the mixture as shown in Example 2 below.

A suitable anti-foaming agent is at least water dispersible, preferably water soluble, and does not contain water itself. The anti-foaming agent may be any known antifoam compound, including, for example a silicone antifoam compound, an alcohol antifoam compound, light petroleum odorless hydrocarbons, fatty acid esters, aliphatic C₁₈-C₄₀ ketones, and nonionic polyhydroxyl derivatives, and any mixture thereof.

Silicone antifoam compounds are defined herein as any antifoam compound including a silicone component. Such silicone antifoam compounds also typically contain a silica component. The term "silicone" as used herein, and in general throughout the industry, encompasses a variety of relatively high molecular weight polymers containing siloxane units and hydrocarbyl group of various types like the polyorganosiloxane oils, such as polydimethyl-siloxane, dispersions or emulsions of polyorganosiloxane oils or resins, and combinations of polyorganosiloxane with silica particles wherein the polyorganosiloxane is chemisorbed or fused onto the silica.

Hydrocarbons are defined herein as including aliphatic, alicyclic, aromatic, and heterocyclic saturated or unsaturated hydrocarbons having from about 12 to about 70 carbon atoms. The term "paraffin" as used in, this discussion, is intended to include mixtures of true paraffins and cyclic hydrocarbons.

The concentration of the antifoaming agent is from about 0.005% to about 5%, preferably from 0.01% to about 3%, and most preferably from about 0.05% to about 2%.

### Water Soluble Coating

Controlling the temperature of the reaction mixture as a function of time is one of the objects of this invention, and control is accomplished largely by coating the exothermic generating particles. While not wanting to be bound by any one theory, it is believed that the coated exothermic generating particles cannot react with the aqueous solution until the coating dissolves. As the coating on the exothermic generating particles begins to dissolve, the exposed particles begin to react and generate heat. The concentration of the water soluble coating material in the reaction mixture is from about 3% to about 70%, preferably from about 5% to about 65%, and more preferably from about 8% to about 60%, by weight, of the reaction mixture.

The water soluble coating of the present invention comprises at least one water soluble coating ingredient consisting of PEG having a molecular weight from 2000-6000, and mixtures thereof. It has been found that PEG with a lower molecular weight that 600 is in a liquid state at room temperature which is not desirable since the exothermic particles migrate to the bottom of the container. This, in turn, delays the start of the exothermic reaction. It has been found that a water soluble coating comprising PEG with molecular weights between 600 and 2000 still demonstrate a tendency of the exothermic reaction particles migrating upon storage. It is known that PEG with a higher molecular weight than 6000 have a melting point above 60°C, and it is believed that the high melting point interferes with the addition of the volatile component in the present invention. If the melting point of the PEG is too high, the volatile component will evaporate upon mixing with the water soluble coating when in liquid form, thereby eliminating the volatile component from the mixture.

The coating can be applied to the exothermic generating particles by any appropriate means. The easiest method is to soften or melt the coating material and mix it with the desired amount of exothermic generating particles.

While it is necessary to coat the exothermic generating particles of the reaction mixture, the volatile component, the anti-foaming agent, the buffer, and the optional components (discussed below), can be coated along with the exothermic generating particles or they can be coated separately from the exothermic generating particles. Combinations of these choices will also produce acceptable results in many cases. Therefore, coating components other than the exothermic generating particles is the prerogative of the formulator.

Additional coating materials may be added in small amounts up to about 10.0% by weight of the water soluble coating, preferably up to about 5.0% by weight of the water soluble coating, and most preferably up to about 2.0%. Please see the incorporated references above for additional coating materials.

### Buffer

The reaction mixtures of the present invention includes as an essential component, a buffer. The buffer can provide a variety of benefits, such as acceleration or deceleration of the exothermic reaction and pH control at the end of the reaction. It has also been found that the level of buffer can delay the appearance time of the precipitation of exothermic particle salts after the reaction is completed.

It is well known that certain exothermic generating particles will react faster than others. A buffer can speed up or slow down a reaction mixture. It is understood, however, that even with a buffer, uncontrolled exothermic reactions will generally follow the time vs. temperature curves depicted in Line "A" of Figure 1. Thus, the buffer works to provide a favorable thermodynamic environment for the reaction mixture, but the buffer does not control the time vs. temperature profile of the reaction. With regard to pH, it is often desirable to control the pH both during the reaction and at the end of the reaction. During the reaction, the pH can contribute to the favorable thermodynamic environment as discussed above, by adjusting the amount of buffer the speed of the reaction can be increased or decreased. The buffer can also regulate the final pH of the reaction mixture when the exothermic reaction is nearing completion. The final pH may be important because at certain pHs the reaction products will precipitate after the completing of the reaction. By increasing the amount of buffer, the appearance of precipitates can be delayed for up to seven days. However, the increase in the amount of buffer must be controlled so as to not increase the rate of reaction significantly. It has been found that a ratio of exothermic particles to buffer of 1:6.5 (w/w) demonstrates a preferred ability to delay the precipitation of exothermic particle salts after the reaction is completed. Regardless, a buffer may help the formulator of the reaction mixtures disclosed herein.

Preferably, if a buffer is present in the reaction mixtures of this invention, the ratio by weight of the exothermic generating particles to the buffer is in the range of from 200:1 to 1:200, preferably from 50:1 to 1:50, and more preferably from 10:1 to 1:10. And the buffer is preferably selected from the group consisting of citric acid, malic acid, fumaric acid, succinic acid, tartaric acid, formic acid, acetic acid, propanoic acid, butyric acid, valeric acid, oxalic acid, malonic acid, glutaric acid, adipic acid, glycolic acid, aspartic acid, pimelic acid, maleic acid, phthalic acid, isophthalic acid, terphthalic acid, glutamic acid, lactic acid, hydroxyl acrylic acid, alpha hydroxyl butyric acid, glyceric acid, tartronic acid, salicylic acid, gallic acid, mandelic acid, tropic acid, ascorbic acid, gluconic acid, cinnamic acid, benzoic acid, phenylacetic acid, nicotinic acid, kainic acid, sorbic acid, pyrrolidone carboxylic acid, trimellitic acid, benzene sulfonic acid, toluene sulfonic acid, potassium dihydrogen phosphate, sodium hydrogen sulfite, sodium dihydrogen phosphate, potassium hydrogen sulfite, sodium hydrogen pyrosulfite, acidic sodium hexametaphosphate, acidic sodium pyrophosphate, acidic potassium pyrophosphate, sulfamic acid, ortho-phosphoric acid, pyro-phosphoric acid and mixtures thereof.

### Exothermic Generating Particles

The exothermic generating particles of the present invention are preferably selected from the group consisting of uncomplexed metals, metal salts, metal oxides, metal hydroxides, metal hydrides and mixtures thereof. The metals are selected from the group consisting of beryllium, magnesium, lithium, sodium, calcium, potassium, iron, copper, zinc, aluminum and mixtures thereof. These particles may also be selected from the group consisting of beryllium hydroxide, beryllium oxide, beryllium oxide monohydrate, lithium aluminum hydride, calcium oxide, calcium hydride, potassium oxide, magnesium chloride, magnesium sulfate, aluminum bromide, aluminum iodide, sodium tetraborate, sodium phosphate and mixtures thereof. The concentration of the exothermic generating particles in the reaction mixture is from about 3% to about 60%, preferably from about 5% to about 55%, and more preferably from about 8% to about 50%, by weight, of the reaction mixture.

It is preferred, although not required, that the exothermic generating particles (without the coating) have an average particle diameter of from about 10 microns to about 1000 microns, preferably from about 100 microns to about 500 microns, and more preferably from about 200 microns to about 400 microns.

### Volatile Component

The reaction mixtures disclosed herein include as an essential component a volatile component that is preferably selected from the group consisting of a perfume, a fragrance, an insect repellent, a fumigant, a disinfectant, a bactericide, an insecticide, a pesticide, a germicide, an acaricide, a sterilizer, a deodorizer, a fogging agent and mixtures thereof. The concentration of volatile component in the reaction mixture is from about 0.01% to about 20%, preferably from about 0.1% to about 15%, and more preferably from about 0.5% to about 10%, by weight, of the reaction mixture.

"Volatile component" as used herein means any compound that is evolved from a reaction mixture according to the present invention to the surrounding environment during an exothermic reaction. The term "volatile" does not imply any restrictions on the vapor pressure or the boiling point of the component. For example, many fine fragrances have boiling points well above the boiling point of water, while other fragrances have boiling points below water. Both types of fragrances fall within the definition of "volatile components" if they are evolved during an exothermic reaction according to the present invention. Necessarily, however, the aqueous solution cannot be considered the volatile component even though a portion of the aqueous solution may evolve during the exothermic reaction.

Fragrances are preferred volatile components for use in the present reaction mixture and preferred fragrances are selected from the group consisting of musk oil, civet, castreum, ambergris, plant perfumes, sandalwood oil, neroli oil, bergamot oil, lemon oil, lavender oil, sage oil, rosemary oil, peppermint oil, eucalyptus oil, menthol, camphor, verbena oil, citronella oil, cauout oil, salvia oil, clove oil, chamomille oil, sandalwood oil, costus oil, labdanum oil, broom extract, carrot seed extract, jasmine extract, minmosa extract, narcissus extract, olibanum, extract, rose extract, acetophenonene, dimethylinadane derivatives, naphthaline derivatives, allyl caprate, .alpha.-amylcinnamic aldehyde, anethole, anisaldehyde, benzyl acetate, benzyl alcohol, benzyl propionate, borneol, cinnamyl acetate, cinnamyl alcohol, citral citronnellal, cumin aldehyde, cyclamen aldehyde, decanol, ethyl butyrate, ethyl caprate, ethyl cinnamate, ethyl vanillin, eugenol, geraniol, exenol, alpha.-hexylcinnamic aldehyde, hydroxycitrofnellal, indole, iso-amyl acetate, iso-amyl iso-valeratek iso-eugenol, linalol, linalyl acetate, p-methylacetophenone, methyl anthranilate, methyl dihydroasmonate, methyl eugenol, methyl-.beta.-naphthol ketone, methylphenhlcarbinyl acetate, musk ketol, musk xylol, 2,5,6-nanodinol, .gamma.-nanolactone, phenylacetoaldehydodimethyl acetate, beta.-phenylethyl alcohol, 3,3,5-trimethylcyclohexanol, gamma.-undecalactone, undecenal, vanillin, and mixtures thereof.

### Aqueous Solution

An optional component of the present reaction mixtures is an aqueous solution. The aqueous solution performs two functions in the reaction mixture. Specifically, it dissolves the water soluble coating on the exothermic particles and then reacts with the exothermic generating particles to generate heat. It is understood that the amount of the aqueous solution is quite flexible. While a sufficient amount of the aqueous solution must be present to dissolve the coating and to react with the exothermic particles, excess aqueous solution is often acceptable and may even be desirable. In fact, excess aqueous solution acts as a heat sink for the reaction system. In this capacity the aqueous solution can, in some circumstances, be used to control the maximum temperature of a given reaction system. The aqueous solution, however, is generally not useful for controlling the time verses temperature curves for the reaction system as described above. Thus, those skilled in the art will be able to select the proper amount of aqueous solution for a given reaction system.

The most common and most preferred aqueous solution is water and solutions containing water. Monohydric alcohols and other low molecular weight liquids are suitable for use in the present invention. The only criterion for an "aqueous solution" is that it dissolves the water soluble coatings described above, and that it react with the chosen exothermic generating particles. The concentration of aqueous solution in the reaction mixture is from about 30% to about 97%, preferably from about 50% to about 95%, and more preferably from about 60% to about 90%, by weight, of the reaction mixture.

### Other Ingredients

The reaction mixtures of the present invention may comprise, as optional components, other ingredients. These optional ingredients can be a thickening agent or a visual enhancement agent.

The use of a thickening agent can be used to further control the rate of the exothermic reaction in the initial 20-30 minutes of the reaction. The initial stage of the reaction is critical to control since the nature of exothermic reactions are to "peak out" early in the reaction, as shown in Figure 1 in reaction "A". Without being limited to a theory, it is believed that a thickener increases the viscosity of the exothermic reaction components and slows the transport of the aqueous solution to the exothermic particles. The concentration of the thickening agent, if present in the reaction mixture, is from about 0.005% to about 5%, preferably from 0.01% to about 3%, and more preferably form about 0.05% to about 2%. A thickening agent should be at least water dispersible and is preferably water soluble, without containing any water itself. A thickening agent may be selected from the group consisting of polyacrylic acids, gums such as xanthan gum, cellulose, ethoxylated cellulose, carboxymethylcellulose, hydroxymethylcellulose, hydroxypropyl cellulose, hydroxyethyl cellulose, clay, silica, and any mixtures thereof. A preferred thickening agent is polyacrylic acid, sold under the tradename CARBOPOL 956 by BFGoodrich.

The visual enhancement agents are selected from the group consisting of a dye, a chemiluminescence agent, a fluorescence agent, a peanescence agent, and mixtures thereof. More preferably, the visual enhancement agent is selected from the group consisting of fire-fly luciferase, adenosinetriphosphate, ethylene glycol disteacate and mixtures thereof. These visual enhancement agents can be used to color the reaction mixture, make it "glow", or provide other visually satisfying effects. The concentration of in the visual enhancement agents, if present in the reaction mixture is from about 0.01% to about 30%, preferably from about 0.1 % to about 20%, and more preferably from about 0.5% to about 15%, by weight, of the reaction mixture.

### Apparatus

In yet another aspect of this invention there is provided an apparatus for generating heat, the apparatus comprises a container and the following reaction components: exothermic generating particles comprising a water soluble coating that encases a portion of the particles wherein at least one water soluble coating ingredient consisting of PEG having a molecular weight from 2000-6000, and mixtures thereof; a volatile component; and a buffer. The apparatus optionally further comprises an aqueous solution. The reaction components for use in the apparatuses of the present invention are the same as those discussed above. The apparatus of the present invention is preferably a self contained and portable device in which an exothermic reaction is conducted. Preferably, the apparatus container should have at least one vent or opening to emit the volatile components that are evolved during the exothermic reaction. Moreover, the container should be constructed of a material that can withstand the maximum temperature of the exothermic reaction. Many materials fulfill this requirement because the maximum temperature of the reaction might be as low as 35°C, higher temperature reaction might require higher temperature tolerance. Glass, plastic, styrofoam, metal, liquid impermeable paper, and many other materials are suitable for use in the present invention. The container is preferably dear, transparent, or translucent, although opaque containers, while less preferable, are suitable for use herein. In the present apparatuses, the exothermic generating particles can be in the form of a dry powder or suspended in a homogenous water soluble coating.

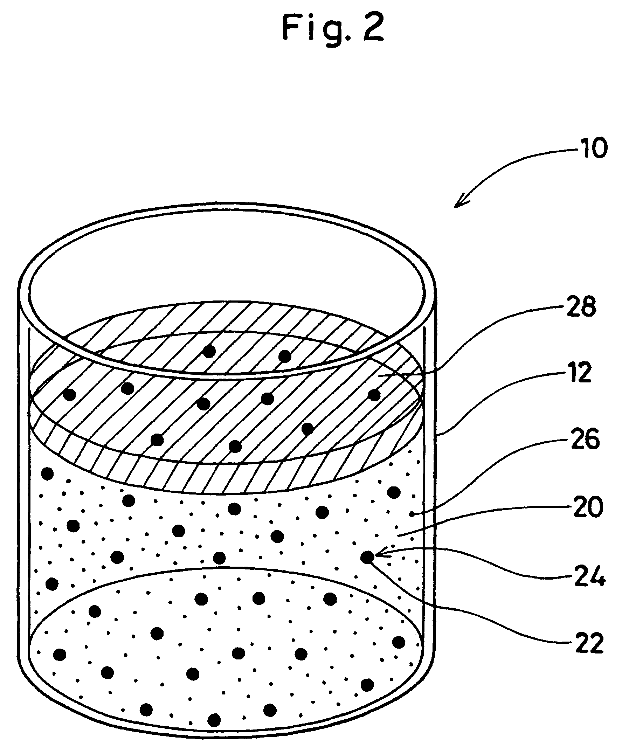

Figure 2 is a schematic representation of an apparatus 10 according to the present invention. Apparatus 10 comprises container 12 and reaction mixture 20, which includes exothermic generating particles 22 with a water soluble coating 24. Reaction mixture 20 further comprises buffer particles 26 and an aqueous solution 28. Volatile component 30 appears throughout reaction mixture 20 as emulsified droplets, although volatile component 30 can also be dissolved in aqueous solution 28 or incorporated into coating 24. Container 12 may sit on a base that houses a light source and a power source (base, light source, and power source not shown).

The reaction mixture used in the apparatuses of the present invention should be controllable as discussed above. That is, when the reaction components are mixed together in the present apparatuses, the reaction mixture should increase in temperature to a Set Temperature that is greater than about 35°C and less than about 75°C, preferably between about 35°C and 60°C, within less than about 20 minutes. Preferably, the reaction mixture within the apparatus remains within 15°C, preferably 10°C of the Set Temperature for at least about 45 minutes, preferably for at least about 60 minutes, and most preferably for at least 80 minutes.

In one preferred embodiment of the present invention, the apparatus includes a light source. The light source, which can optionally provide colored light, can be used to enhance the visual effect of the apparatus. Moreover, as discussed above, visual enhancement agents may be employed in the reaction mixture in addition to the light source. The light source can be used to accentuate the visual enhancement agents, or simply to "light up" the apparatus. The light source can be battery powered, solar powered or the like. While generally not preferred, the light source could be externally powered by, for example, an electrical outlet. The apparatuses of the present invention are preferably portable, thus using external power may limit the portability. The light source can be within the container, or adjacent the exterior of the container. If the light source is placed in the container, it will be preferable to encase the light source and its power supply in a liquid impermeable barrier to shield the device from the aqueous solution. Preferably, the container sits on a base that both supports the container, and provides a housing for the light source.

The light source may contribute some heat to the reaction mixture, but that is not the desired function. Moreover, most battery operated devices operated at low voltage, and produce very little heat. Thus the light source is not intended to function as a control mechanism.

One especially preferred light source for use in the present apparatuses is a light emitting diode ("LED"). LEDs are well known to the art and examples of these devices can be found in, for example, US Patent No. 5,963,185, which issued to Havel on October 5, 1999, and US Patent No. 5,940,683, which issued to Holm, et al. on August 17, 1999. The entire disclosure of the Havel and Holm et al. patents are incorporated herein by reference. LEDs are small devices that provide numerous colors from a single source. Thus, from one device, a variety of colors can be projected onto the reaction mixture increasing the range of available visual effects. These devices have the additional benefit in that they operate at low power, and would require only a small battery or solar power cell.

### EXAMPLES

The following examples illustrate the reaction mixtures of the present invention, but are not necessarily meant to limit or otherwise define the scope of the invention.

### Example 1

### Method of Coating the Exothermic Generating Particles

Exothermic generating particles are coated with polyethylene glycol (PEG) as follows. First, add PEG 2000 into a glass beaker and heated until melted which is around 50°C ± 5°C. Next, add magnesium slowly to the PEG melt with mechanical stirring keeping the mixture at 50°C ± 5°C. Third, add citric acid slowly to the mixture, continuing the mechanical stirring and keeping the mixture at 50°C ± 5°C. Finally, add the volatile component to the mixture and continuously stir and keep the mixture at 50°C ± 5°C for approximately 10 minutes. Add the mixture to a plastic cup and tap gently to make the mixture even. Immediately seal the cup with film and keep at -10°C for at least 60 minutes.

### Example 2

Table 3 below demonstrates the height of the resulting foam in three difference formulations that have been stored for three days at 40°C/ 75% RH. Formula A does not contain any anti-foaming component and foam appears within the first five minutes and continues to increase in height until its peak of 15 mm after 13 minutes. Formulas B and C both contain the anti-foaming component, here the anti-foaming agent used comprises 4.25% polydimethylsiloxane, 0.75% SiO₂, and 95% PEG8000 sold under the tradename BLUE AE COFLAKE by Heterene. The result of adding the anti-foaming agent is to delay the appearance of foam for 25 minutes and to decrease the maximum height of the foam to 2 mm or less.

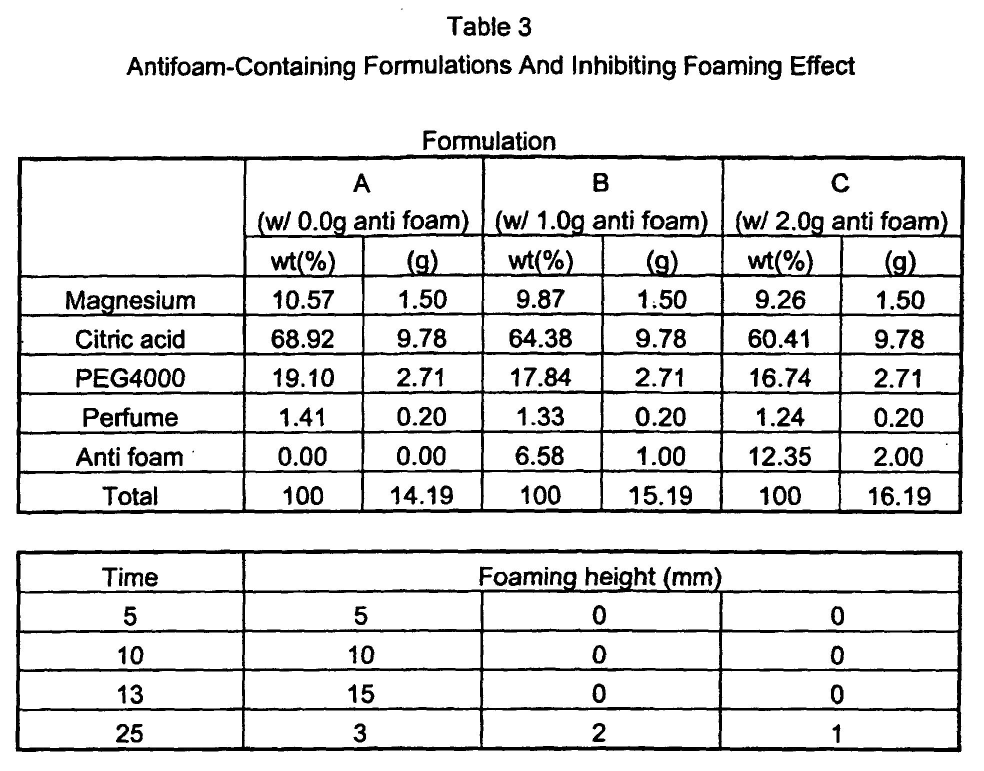

The concentration of the anti-foaming agent should be limited so the rate of reaction is not decreased, as in the case of the anti-foaming agent used in Table 3 which is believed to occur due to the high percentage of PEG8000. The concentration of the anti-foaming agent should also be limited so as to not leave a residue at the end of the reaction. The concentration of the antifoaming agent is from about 0.005% to about 5%, preferably from 0.01% to about 3%, and most preferably form about 0.05% to about 2%.

### Example 3

**Table 4**

| Continuous Layer Formulations | | | | | | |
|---|---|---|---|---|---|---|
| | PEG 2000 | | PEG 4000 | | PEG 2000/4000 | |
| | (g) | (w%) | (g) | (w%) | (g) | (w%) |
| Magnesium | 1.50 | 10.57 | 1.50 | 10.57 | 1.50 | 10.57 |
| Citric acid | 9.78 | 68.92 | 9.78 | 68.92 | 9.78 | 68.92 |
| PEG2000 | 2.71 | 19.10 | - | - | 1.355 | 9.55 |
| PEG4000 | - | - | 2.71 | 19.10 | 1.355 | 9.55 |
| Perfume | 0.20 | 1.41 | 0.20 | 1.41 | 0.20 | 1.41 |
| Total | 14.19 | 100.00 | 14.19 | 100.00 | 14.19 | 100.00 |
| Migration of exothermic particles at 50°C/1 day | No | | No | | No | |
| Time to reach target temperature of 45°C | 16 minutes | | 12 minutes | | 14 minutes | |

The present invention improves upon the time required to reach a target temperature by increasing the molecular weight of PEG so that a continuous layer system can be used. The higher molecular weight PEG gives a more viscous medium where the exothermic particles are hindered from migrating during storage. The present invention, demonstrated in the formulations in Table 4, was stored for 24-36 hours at 50°C. The particle migration shown by 2-layer systems does not occur and the reaction of the present invention reaches Set Temperature within 20 minutes.

## Claims

1. An exothermic reaction mixture comprising the following reaction components:
a) exothermic generating particles;
b) a volatile component;
c) an anti-foaming agent; and
d) a buffer;
wherein the reaction mixture is suspended in a continuous phase water soluble coating comprising at least one water soluble coating ingredient consisting of PEG having a molecular weight from 2000-6000, and mixtures thereof.

2. The reaction mixture of claim 1, wherein the reaction components further comprise an aqueous solution.

3. The reaction mixture of claim 2, wherein when the reaction components are mixed together, the temperature of the reaction mixture increases to a Set Temperature that is greater than about 35°C and less than about 75°C, preferably between about 35°C and 60°C, within less than about 30 minutes, preferably within less than about 20 minutes.

4. The reaction mixture of claim 3, wherein the reaction mixture remains within 15°C, preferably within 10°C, of the Set Temperature for at least about 45 minutes, preferably for at least about 60 minutes, most preferably 80 minutes.

5. The reaction mixture of claim 1, wherein the volatile component is selected from the group consisting of a perfume, a fragrance, an insect repellent, a fumigant, a disinfectant, a bactericide, an insecticide, a pesticide, a germicide, an acaricide, a sterilizer, a deodorizer, a fogging agent and mixtures thereof.

6. The reaction mixture of claim 1, wherein the volatile component is selected from the group consisting of a musk oil, civet, castreum, ambergris, plant perfumes, sandalwood oil, neroli oil, bergamot oil, lemon oil, lavender oil, sage oil, rosemary oil, peppermint oil, eucalyptus oil, menthol, camphor, verbena oil, citronella oil, cauout oil, salvia oil, clove oil, chamomille oil, sandalwood oil, costus oil, labdanum oil, broom extract, carrot seed extract, jasmine extract, minmosa extract, narcissus extract, olibanum, extract, rose extract, acetophenonene, dimethylinadane derivatives, naphthaline derivatives, allyl caprate, .alpha.-amylcinnamic aldehyde, anethole, anisaldehyde, benzyl acetate, benzyl alcohol, benzyl propionate, borneol, cinnamyl acetate, cinnamyl alcohol, citral citronnellal, cumin aldehyde, cyclamen aldehyde, decanol, ethyl butyrate, ethyl caprate, ethyl cinnamate, ethyl vanillin, eugenol, geraniol, exenol, alpha.-hexylcinnamic aldehyde, hydroxycitrolnellal, indole, iso-amyl acetate, iso-amyl iso-valeratek iso-eugenol, linalol, linalyl acetate, p-methylacetophenone, methyl anthranilate, methyl dihydroasmonate, methyl eugenol, methyl-.beta.-naphthol ketone, methylphenhlcarbinyl acetate, musk ketol, musk xylol, 2,5,6-nanodinol, .gamma.-nanolactone, phenylacetoaldehydodimethyl acetate, beta.-phenylethyl alcohol, 3,3,5-trimethylcyclohexanol, .gamma.-undecalactone, undecenal, vanillin, and mixtures thereof.

7. The reaction mixture of claim 1, wherein the exothermic generating particles are selected from the group consisting of uncornplexed metals, metal salts, metal oxides, metal hydroxides, metal hydrides and mixtures thereof, wherein the metals are selected from the group consisting of beryllium, magnesium, lithium, sodium, calcium, potassium, iron, copper, zinc, aluminum and mixtures thereof.

8. The reaction mixture of claim 7, wherein the exothermic generating particles are selected from the group consisting of beryllium hydroxide, beryllium oxide, beryllium oxide monohydrate, lithium aluminum hydride, calcium oxide, calcium hydride, potassium oxide, magnesium chloride, magnesium sulfate, aluminum bromide, aluminum iodide, sodium tetraborate, sodium phosphate and mixtures thereof.

9. The reaction mixture of claim 1, wherein the exothermic generating particles have an average particle diameter of from about 10 microns to about 1000 microns, preferably from about 100 microns to about 500 microns, and more preferably from about 200 microns to about 400 microns.

10. The reaction mixture of claim 1, wherein the buffer is selected from the group consisting of citric acid, malic, acid, fumaric acid, succinic acid, tartaric acid, formic acid, acetic acid, propanoic acid, butyric acid, valeric acid, oxalic acid, malonic acid, glutaric acid, adipic acid, glycolic acid, aspartic acid, pimelic acid, maleic acid, phthalic acid, isophthalic acid, terphthalic acid, glutamic acid, lactic acid, hydroxyl acrylic acid, alpha hydroxyl butyric acid, glyceric acid, tartronic acid, salicylic acid, gallic acid, mandelic acid, tropic acid, ascorbic acid, gluconic acid, cinnamic acid, benzoic acid, phenylacetic acid, nicotinic acid, kainic acid, sorbic acid, pyrrolidone carboxylic acid, trimellitic acid, benzene sulfonic acid, toluene sulfonic acid, potassium dihydrogen phosphate, sodium hydrogen sulfite, sodium dihydrogen phosphate, potassium hydrogen sulfite, sodium hydrogen pyrosulfite, acidic sodium hexametaphosphate, acidic sodium pyrophosphate, acidic potassium pyrophosphate, sulfamic acid, ortho-phosphoric acid, pyro-phosphoric acid and mixtures thereof.

11. The reaction mixture of claim 1, wherein the ratio by weight of the exothermic generating particles to the buffer is in the range of from 200:1 to 1:200, preferably from 50:1 to 1:50, and more preferably from 10:1 to 1:10.

12. The reaction mixture of claim 1, where the anti-foam agent is selected from the group consisting of silicone antifoam compound, an alcohol antifoam compound, light petroleum odorless hydrocarbons, fatty acid esters, fatty acid esters of monovalent alcohols, aliphatic C18-C40 ketones, nonionic polyhydroxyl derivatives and mixtures thereof.

13. The reaction mixture of claim 1, further comprising a thickening agent selected from the group consisting of polyacrylic acids, gums, cellulose, thoxylated cellulose, carboxymethylcellulose, hydroxymethylcellulose, hydroxypropyl cellulose, hydroxyethyl cellulose, clay, silica, and any mixtures thereof.

14. The reaction mixture of claim 1, further comprising a water soluble visual enhancement agent selected from the group consisting of a dye, a chemiluminescence agent, a fluorescence agent, a pearlescence agent, and mixtures thereof.

15. The reaction mixture of claim 14, wherein the visual enhancement agent is selected from the group consisting of fire-fly luciferase, adenosinetriphosphate, ethylene glycol disteate and mixtures thereof.

16. A process for generating heat through a continuous phase system, the process comprising the steps of:
a) providing exothermic generating particles coated with a water soluble coating that entirely encases the exothermic generating particles, wherein the water soluble coating consists of PEG having a molecular weight from 2000-6000, and mixtures thereof;
b) providing a volatile component, an anti-foaming agent, and a buffer; and
c) adding the coated exothermic generating particles, the volatile component, the anti-foaming agent, and the buffer into an aqueous solution.

17. The process of claim 16, step (b) further comprising the step of providing a thickening agent.

18. An apparatus for generating heat comprising a container and the following reaction components:
a) a water soluble coating entirely enclosing exothermic generating particles wherein the water soluble coating consists of PEG having a molecular weight from 2000-6000, and mixtures thereof;
b) a volatile component;
c) an anti-foaming agent; and
d) a buffer.

19. The apparatus of claim 18, wherein the reaction components further comprise an aqueous solution.

20. The apparatus of claim 19, wherein a reaction mixture is created when the reaction components are mixed together, and the temperature of the reaction mixture increases to a Set Temperature that is greater than about 35°C and less than about 75°C, preferably between about 35°C and 60°C, within at least 30 minutes, preferably within at least about 20 minutes.

21. The apparatus of claim 20, wherein the reaction mixture remains within 10 °C of the Set Temperature for at least about 45 minutes, preferably for at least about 60 minutes, most preferably for at least about 80 minutes.

22. The apparatus of claim 18, further comprising a light emitting device capable of emitting a variety of colors, preferably a light emitting diode.

## Patentansprüche

1. Exotherme Reaktionsmischung umfassend die folgenden Reaktionskomponenten:
a) Wärme erzeugende Teilchen;
b) flüchtige Komponente;
c) Schaumverhinderer und
d) Puffer,
wobei die Reaktionsmischung suspendiert ist in einer wasserlöslichen Beschichtung mit kontinuierlicher Phase, umfassend mindestens einen wasserlöslichen Bestandteil der Beschichtung bestehend aus PEG mit einem Molekulargewicht von 2000 bis 6000 und Mischungen davon.

2. Reaktionsmischung aus Anspruch 1, wobei die Reaktionskomponenten weiterhin umfassen eine wässrige Lösung.

3. Reaktionsmischung aus Anspruch 2, wobei, wenn die Reaktionskomponenten miteinander vermischt werden, die Temperatur der Reaktionsmischung innerhalb von weniger als 30 Minuten, vorzugsweise innerhalb von weniger als 20 Minuten, auf eine Solltemperatur ansteigt, die höher als ungefähr 35 °C und niedriger als ungefähr 75 °C ist, vorzugsweise zwischen ungefähr 35 °C und 60 °C.

4. Reaktionsmischung aus Anspruch 3, wobei die Reaktionsmischung mindestens ungefähr 45 Minuten lang, vorzugsweise ungefähr 60 Minuten lang, am meisten bevorzugt ungefähr 80 Minuten lang, nicht mehr als 15 °C, vorzugsweise nicht mehr als 10 °C, von der Solltemperatur abweicht.

5. Reaktionsmischung aus Anspruch 1, wobei die flüchtige Komponente ausgewählt ist aus der Gruppe bestehend aus einem Duftstoff, einem Riechstoff, einem Insektenschutzmittel, einem Räuchermittel, einem Desinfektionsmittel, einem Bakterizid, einem Insektizid, einem Pestizid, einem Germizid, einem Akarizid, einem desodorierenden Mittel, einem nebelbildenden Mittel und Mischungen davon.

6. Reaktionsmischung aus Anspruch 1, wobei die flüchtige Komponente ausgewählt ist aus der Gruppe bestehend aus Moschusöl, Zibet, Bibergeil, Ambra, Pflanzenduftstoffen, Sandelholzöl, Neroliöl, Bergamottöl, Zitronenöl, Lavendelöl, Salbeiöl, Rosmarinöl, Pfefferminzöl, Eukalyptusöl, Menthol, Kampfer, Verbenenöl, Citronellöl, Cauoutöl, Salbeiöl, Gewürznelkenöl, Kamillenöl, Sandelholzöl, Costusöl, Labdanumöl, Ginsterextrakt, Karottensamenextrakt, Jasminextrakt, Mimosenextrakt, Narzissenextrakt, Olibanumextrakt, Rosenextrakt, Acetophenonen, Dimethylindanderivaten, Naphthalinderivaten, Allylcaprat, alpha-Amylzimtaldehyd, Anethol, Anisaldehyd, Benzylacetat, Benzylalkohol, Benzylpropionat, Bomeol, Cinnamylacetat, Cinnamylalkohol, Citralcitronnellal, Cuminaldehyd, Cyclamenaldehyd, Decanol, Ethylbutyrat, Ethylcaprat, Ethylcinnamat, Ethylvanillin, Eugenol, Geraniol, Hexenol, alpha-Hexylzimtaldehyd, Hydroxycitronellal, Indol, Isoamylacetat, Isoamyl-isovaleratisoeugenol, Linalol, Linalylacetat, p-Methylacetophenon, Methylanthranilat, Methyldihydrojasmonat, Methyleugenol, Methyl-beta-naphtholketon, Methylphenylcarbinylacetat, Moschusketon, Moschusxylol, 2,5,6-Nanodinol, gamma-Nonalacton, Phenylacetaldehyd-dimethylacetat, beta-Phenylethylalkohol, 3,3,5-Trimethylcyclohexanol, gamma-Undecalacton, Undecenal, Vanillin und Mischungen davon.

7. Reaktionsmischung aus Anspruch 1, wobei die Wärme erzeugenden Teilchen ausgewählt sind aus der Gruppe bestehend aus nicht komplexierten Metallen, Metallsalzen, Metalloxiden, Metallhydroxiden, Metallhydriden und Mischungen davon, wobei die Metalle ausgewählt sind aus der Gruppe bestehend aus Beryllium, Magnesium, Lithium, Natrium, Calcium, Kalium, Eisen, Kupfer, Zink, Aluminium und Mischungen davon.

8. Reaktionsmischung aus Anspruch 7, wobei die Wärme erzeugenden Teilchen ausgewählt sind aus der Gruppe bestehend aus Berylliumhydroxid, Berylliumoxid, Berylliumoxid-Monohydrat, Lithiumaluminiumhydrid, Calciumoxid, Calciumhydrid, Kaliumoxid, Magnesiumchlorid, Magnesiumsulfat, Aluminiumbromid, Aluminiumiodid, Natriumtetraborat, Natriumphosphat und Mischungen davon.

9. Reaktionsmischung aus Anspruch 1, wobei die Wärme erzeugenden Teilchen einen durchschnittlichen Teilchendurchmesser von ungefähr 10 µm bis ungefähr 1000 µm, vorzugsweise von ungefähr 100 µm bis ungefähr 500 µm, und mehr bevorzugt von ungefähr 200 µm bis ungefähr 400 µm aufweisen.

10. Reaktionsmischung aus Anspruch 1, wobei der Puffer ausgewählt ist aus der Gruppe bestehend aus Zitronensäure, Apfelsäure, Fumarsäure, Bernsteinsäure, Weinsäure, Ameisensäure, Essigsäure, Propionsäure, Buttersäure, Valeriansäure, Oxalsäure, Malonsäure, Glutarsäure, Adipinsäure, Glycolsäure, Asparaginsäure, Pimelinsäure, Maleinsäure, Phthalsäure, Isophthalsäure, Terephtalsäure, Glutaminsäure, Milchsäure, Hydroxyacrylsäure, alpha-Hydroxybuttersäure, Glycerinsäure, Tatronsäure, Salicylsäure, Gallussäure, Mandelsäure, Tropasäure, Ascorbinsäure, Gluconsäure, Zimtsäure, Benzoat, Phenylessigsäure, Nicotinsäure, Kaininsäure, Sorbinsäure, Pyrrolidon-Carbonsäure, Trimelinsäure, Benzolsulfonsäure, Toluolsulfonsäure, Kaliumdihydrogenphosphat, Natriumhydrogensulfit, Natriumdihydrogenphosphat, Kaliumhydrogensulfit, Natriumhydrogenpyrosulfit, saurem Natriumhexametaphosphat, saurem Natriumpyrophosphat, saurem Kaliumpyrosulfat, Sulfaminsäure, ortho-Phosphorsäure, Pyrophosphorsäure und Mischungen davon.

11. Reaktionsmischung aus Anspruch 1, wobei das Gewichtsverhältnis zwischen den Wärme erzeugenden Teilchen und dem Puffer im Bereich liegt von 200:1 bis 1:200, vorzugsweise von 50:1 bis 1:50, und mehr bevorzugt von 10:1 bis 1:10.

12. Reaktionsmischung aus Anspruch 1, wobei der Schaumverhinderer ausgewählt ist aus der Gruppe bestehend aus silikonhaltiger, Schaum unterdrückender Verbindung, einer alkoholhaltigen, Schaum unterdrückenden Verbindung, geruchfreien Leichtpetroleum-Kohlenwasserstoffen, Fettsäureestern, Fettsäureestern einwertiger Alkohole, aliphatischen C18-C40-Ketonen, nichtionischen Polyhydroxyderivaten und Mischungen davon.

13. Reaktionsmischung aus Anspruch 1, ferner umfassend ein Verdickungsmittel ausgewählt aus der Gruppe bestehend aus Polyacrylsäuren, Gummistoffen, Cellulose, ethoxylierter Cellulose, Carboxymethylcellulose, Hydromethylcellulose, Hydroxypropylcellulose, Hydroxyethylcellulose, Ton, Kieselerde und Mischungen davon.

14. Reaktionsmischung aus Anspruch 1, ferner umfassend ein wasserlösliches Mittel zur optischen Verbesserung ausgewählt aus der Gruppe bestehend aus einem Farbstoff, einem Chemilumineszenzmittel, einem Fluoreszenzmittel, einem Perlglanzmittel und Mischungen davon.

15. Reaktionsmischung aus Anspruch 14, wobei das Mittel zur optischen Verbesserung ausgewählt ist aus der Gruppe bestehend aus Glühwürmchen-Luziferase, Adenosintriphosphat, Ethylenglycoldistearat und Mischungen davon.

16. Verfahren zur Erzeugung von Wärme mittels eines Systems mit kontinuierlicher Phase, das Verfahren umfassend die Schritte des:
a) Bereitstellens Wärme erzeugender Teilchen beschichtet mit einer wasserlöslichen Beschichtung, welche die Wärme erzeugenden Teilchen vollständig umhüllt, wobei die wasserlösliche Beschichtung besteht aus PEG aufweisend ein Molekulargewicht von 2000 bis 6000 und Mischungen davon;
b) Bereitstellens einer flüchtigen Komponente, eines Schaumverhinderers und eines Puffers, und
c) des Hinzufügens der beschichteten Wärme erzeugenden Teilchen, der flüchtigen Komponente, des Schaumverhinderers und des Puffers in eine wässrige Lösung.

17. Verfahren aus Anspruch 16, Schritt (b), ferner umfassend den Schritt des Bereitstellens eines Verdickungsmittels.

18. Vorrichtung zum Erzeugen von Wärme umfassend einen Behälter und die folgenden Reaktionskomponenten:
a) eine wasserlösliche Beschichtung vollständig einschließend Wärme erzeugende Teilchen, wobei die wasserlösliche Beschichtung besteht aus PEG aufweisend ein Molekulargewicht von 2000 bis 6000 und Mischungen davon;
b) flüchtige Komponente;
c) Schaumverhinderer und
d) Puffer.

19. Vorrichtung aus Anspruch 18, wobei die Reaktionskomponenten ferner umfassen eine wässrige Lösung.

20. Vorrichtung aus Anspruch 19, wobei eine Reaktionsmischung geschaffen wird, wenn die Reaktionskomponenten vermischt werden, und die Temperatur der Reaktionsmischung steigt innerhalb von mindestens 30 Minuten, vorzugsweise innerhalb von mindestens ungefähr 20 Minuten, auf eine Solltemperatur an, die höher ist als ungefähr 35 °C und niedriger als ungefähr 75 °C, vorzugsweise zwischen ungefähr 35 °C und 60 °C.

21. Vorrichtung aus Anspruch 20, wobei die Reaktionsmischung mindestens ungefähr 45 Minuten lang, vorzugsweise mindestens ungefähr 60 Minuten lang, am meisten bevorzugt mindestens ungefähr 80 Minuten lang, nicht mehr als 10 °C von der Solltemperatur abweicht.

22. Vorrichtung aus Anspruch 18, ferner umfassend eine Licht aussendende Vorrichtung, welche in der Lage ist, eine Vielzahl von Farben auszusenden, vorzugsweise eine Leuchtdiode.

## Revendications

1. Mélange réactionnel exothermique comprenant les composants réactionnels suivants:
a) des particules génératrices de chaleur;
b) un composant volatil;
c) un agent anti-mousse; et
d) un agent tampon;
dans lequel le mélange réactionnel est mis en suspension dans un enrobage hydrosoluble en phase continue comprenant au moins un ingrédient d'enrobage hydrosoluble constitué de PEG ayant un poids moléculaire de 2000 à 6000 et de ses mélanges.

2. Mélange réactionnel selon la revendication 1, dans lequel les composants réactionnels comprennent en outre une solution aqueuse.

3. Mélange réactionnel selon la revendication 2, dans lequel, lorsque les composants réactionnels sont mélangés ensemble, la température du mélange réactionnel augmente à une température de consigne qui est supérieure à environ 35° C et inférieure à environ 75° C, de préférence entre environ 35° C et 60° C, en moins d'environ 30 minutes, de préférence en moins d'environ 20 minutes.

4. Mélange réactionnel selon la revendication 3, dans lequel le mélange réactionnel reste dans les 15° C, de préférence dans les 10° C, de la température de consigne pendant au moins environ 45 minutes, de préférence pendant au moins environ 60 minutes, plus préférablement pendant 80 minutes.

5. Mélange réactionnel selon la revendication 1, dans lequel le composant volatil est choisi dans le groupe constitué d'un parfum, d'une fragrance, d'un produit insectifuge, d'un agent fumigène, d'un désinfectant, d'un bactéricide, d'un insecticide, d'un pesticide, d'un germicide, d'un acaricide, d'un stérilisant, d'un déodorant, d'un agent de voile et de leurs mélanges.

6. Mélange réactionnel selon la revendication 1, dans lequel le composant volatil est choisi dans le groupe constitué des suivants: l'huile de musc, la civette, le castreum, l'ambre gris, les parfums végétaux, l'essence de bois de santal, l'essence de néroli, l'essence de bergamote, l'essence de citron, l'essence de lavande, l'essence de sauge, l'essence de romarin, l'essence de menthe poivrée, l'essence d'eucalyptus, le menthol, le camphre, l'essence de verveine, l'essence de citronnelle, l'essence de cauout, l'essence de salvia, l'essence de clou de girofle, l'essence de camomille, l'essence de bois de santal, l'essence de costus, l'essence de labdanum, l'extrait de genêt, l'extrait de semences de carottes, l'extrait de jasmin, l'extrait de mimosa, l'extrait de narcisse, l'extrait d'olibanum, l'extrait de rose, l'acétophénonène, les dérivés de diméthylnadane, les dérivés de naphtaline, le caprate d'allyle, l'aldéhyde a lpha-amylcinnamique, l'anéthol, l'anisaldéhyde, l'acétate de benzyle, l'alcool benzylique, le propionate de benzyle, le bornéol, l'acétate de cinnamyle, l'alcool de cinnamyle, le citral, le citronnellal, l'aldéhyde de cumin, l'aldéhyde de cyclamen, le décanol, le butyrate d'éthyle, le caprate d'éthyle, le cinnamate d'éthyle, l'éthylvanilline, l'eugénol, le géraniol, l'exénol, l'aldéhyde alpha-hexycinnamique, l'hydrosycitronellal, l'indole, l'acétate d'isoamyle, l'isovalérate d'isoamyle, l'isoeugénol, le linalol, l'acétate de linalyle, la p-méthylacétophénone, l'anthranilate de méthyle, le dihydrojasmonate de méthyle, l'eugénol de méthyle, la méthyl-bêta-naphtolcétone, l'acétate de méthylphényicarbinyle, le cétol de musc, le xylol de musc, le 2,5,6-nanodinol, la gamma-nanolactone, l'acétate de phénylacétoaldehydodiméthyle, l'alcool de bêta-phényléthyle, le 3,3,5-triméthylcyclohexanol, la gamma-undécalactone, l'undécénal, la vanille et leurs mélanges.

7. Mélange réactionnel selon la revendication 1dans lequel les particules génératrices de chaleur de la présente invention sont de préférence choisies dans le groupe constitué des métaux non complexés, de sels de métaux, d'oxydes de métaux, d'hydroxydes de métaux, d'hydrures de métaux et de leurs mélanges dans lequel les métaux sont choisis dans le groupe constitué du béryllium, du magnésium, du lithium, du sodium, du calcium, du potassium, du fer, du cuivre, du zinc, de l'aluminium et de leurs mélanges.

8. Mélange réactionnel selon la revendication 7 dans lequel les particules génératrices de chaleur sont choisies dans le groupe constitué de l'hydroxyde de béryllium, de l'oxyde de béryllium, de l'oxyde de béryllium monohydraté, de l'hydrure de lithium-aluminium, de l'oxyde de calcium, de l'hydrure de calcium, de l'oxyde de potassium, du chlorure de magnésium, du sulfate de magnésium, du bromure d'aluminium, de l'iodure d'aluminium, du tétraborate de sodium, du phosphate de sodium et de leurs mélanges.

9. Mélange réactionnel selon la revendication 1, dans lequel les particules génératrices de chaleur ont un diamètre particulaire moyen d'environ 10 micromètres à environ 1000 micromètres, de préférence d'environ 100 micromètres à environ 500 micromètres, mieux encore de 200 micromètres à environ 400 micromètres.

10. Mélange réactionnel selon la revendication 1, dans lequel le tampon est choisi dans le groupe constitué de l'acide citrique, de l'acide maléique, de l'acide fumarique, de l'acide succinique, de l'acide tartrique, de l'acide formique, de l'acide acétique, de l'acide propanoïque, de l'acide butyrique, de l'acide valérique, de l'acide oxalique, de l'acide malonique, de l'acide glutarique, de l'acide adipique, de l'acide glycolique, de l'acide astartique, de l'acide pimélique, de l'acide maléique, de l'acide phtalique, de l'aide isophtalique, de l'acide téréphtalique, de l'acide glutamique, de l'acide lactique, de l'acide hydroxyacrylique, de l'acide alpha hydroxybutyrique, de l'acide glycérique, de l'acide tartronique, de l'acide salicylique, de l'acide gallique, de l'acide mandélique, de l'acide tropique, de l'acide ascorbique, de l'acide gluconique, de l'acide cinnamique, de l'acide benzoïque, de l'acide phénylacétique, de l'acide nicotinique, de l'acide kaïnique, de l'acide sorbique, de l'acide pyrrolidonecarboxylique, de l'acide trimellitique, de l'acide benzènesulfonique, de l'acide toluènesulfonique, du dihydrogénophosphate de potassium, de l'hydrogénosulfite de sodium, du dihydrogénophosphate de sodium, de l'hydrogénosulfite de potassium, de l'hydrogénopyrosulfite de sodium, de l'hexamétaphosphate de sodium acide, du pyrophosphate de sodium acide, du pyrophosphate de potassium acide, de l'acide sulfamique, de l'acide ortho-phosphorique, de l'acide pyrophosphorique et de leurs mélanges.

11. Mélange réactionnel selon la revendication 1, dans lequel le rapport en poids des particules exothermiques génératrices de chaleur au tampon se situe dans la plage d'environ 200:1 à 1:200, de préférence de 50:1 à 1:50, plus préférablement de 10:1 à 1:10.

12. Mélange réactionnel selon la revendication 1, dans lequel l'agent anti-mousse est choisi dans le groupe constitué d'un composé anti-mousse siliconé, d'un composé anti-mousse à base d'alcool, d'hydrocarbures inodores à base de pétrole léger, d'esters d'acides gras, de cétones aliphatiques en C₁₈ à C₄₀ et de dérivés polyhydroxylés non ioniques et de leurs mélanges.

13. Mélange réactionnel selon la revendication 1, comprenant en outre un agent épaississant choisi dans le groupe constitué des acides polyacryliques, des gommes, de la cellulose, de la cellulose éthoxylée, de la carboxyméthylcellulose, de l'hydroxyméthylcellulose, de l'hydroxypropyl cellulose, de l'hydroxyéthylcelluloe, de l'argile, de la silice et de leurs mélanges quelconques.

14. Mélange réactionnel selon la revendication 1, comprenant en outre un agent d'amélioration de l'aspect visuel hydrosoluble choisi dans le groupe constitué d'un colorant, d'un agent de chimioluminescence, d'un agent de fluorescence, d'un agent de perlescence et de leurs mélanges.

15. Mélange réactionnel selon la revendication 14, dans lequel l'agent d'amélioration de l'aspect visuel est choisi dans le groupe constitué d'une luciférase de luciole, de triphosphate d'adénosine, de distéarate d'éthylèneglycol et de leurs mélanges.

16. Procédé de génération de chaleur par un système en phase continue, le procédé comprenant les étapes suivantes:
a) on met en oeuvre des particules génératrices de chaleur revêtues d'un enrobage hydrosoluble qui enrobe entièrement les particules génératrices de chaleur, lequel enrobage hydrosoluble est constitué de PEG ayant un poids moléculaire de 2000 à 6000 et de ses mélanges;
b) on met en oeuvre un composant volatil, un agent anti-mousse et un tampon; et
c) on ajoute les particules génératrices de chaleur enrobées, le composant volatil, l'agent anti-mousse et le tampon dans une solution aqueuse.

17. Procédé selon la revendication 16, dans lequel l'étape (b) comprend par ailleurs l'étape de mise en oeuvre d'un agent épaississant.

18. Appareil de génération de chaleur comprenant un récipient et les composants réactionnels suivants:
a) un enrobage hydrosoluble enrobant entièrement des particules génératrices de chaleur, dans lequel l'enrobage hydrosoluble est constitué de PEG ayant un poids moléculaire de 2000 à 6000 et de ses mélanges;
b) un composant volatil;
c) un agent anti-mousse; et
d) un agent tampon

19. Appareil selon la revendication 18, dans lequel les composants réactionnels comprennent par ailleurs une solution aqueuse.

20. Appareil selon la revendication 19, dans lequel un mélange réactionnel est crée lorsque les composants réactionnels sont mélangés l'un à l'autre et que la température du mélange réactionnel augmente à une température de consigne qui est supérieure à environ 35° C et inférieure à environ 75° C, de préférence entre environ 35° C et 60° C, dans les au moins 30 minutes, de préférence dans les au moins environ 20 minutes.

21. Appareil selon la revendication 20, dans lequel le mélange réactionnel reste dans les 10° C de la température de consigne pendant au moins environ 45 minutes, de préférence pendant au moins environ 60 minutes, plus préférablement pendant au moins environ 80 minutes.

22. Appareil selon la revendication 18, comprenant par ailleurs un dispositif photoémetteur capable d'émettre une variété de couleurs, de préférence une diode photoémettrice.
